(19) **Europäisches Patentamt**
**European Patent Office**
**Office européen des brevets**

(11) **EP 2 910 943 B1**

(12) **EUROPEAN PATENT SPECIFICATION**

(45) Date of publication and mention
of the grant of the patent:
**10.11.2021  Bulletin 2021/45**

(51) Int Cl.:
*G01N 30/88* (2006.01)     *B01D 15/08* (2006.01)
*B01J 20/281* (2006.01)   *B01J 39/04* (2017.01)
*B01J 39/26* (2006.01)     *C07K 1/18* (2006.01)
*C07K 16/00* (2006.01)     *B01D 15/36* (2006.01)
*C08F 222/38* (2006.01)   *G01N 30/96* (2006.01)
*B01J 39/17* (2017.01)

(21) Application number: **13846286.6**

(22) Date of filing: **25.09.2013**

(86) International application number:
**PCT/JP2013/075941**

(87) International publication number:
**WO 2014/061411 (24.04.2014 Gazette 2014/17)**

(54) **CATION EXCHANGE CHROMATOGRAPHY CARRIER FOR REFINING OF ANTIBODIES AND ITS USE AND METHOD FOR SEPARATION OF ANTIBODY MONOMERS FROM AGGREGATES THEREOF**

KATIONENAUSTAUSCHCHROMATOGRAFIETRÄGER ZUM RAFFINIEREN VON ANTIKÖRPERN UND VERFAHREN ZUR TRENNUNG VON ANTIKÖRPER-MONOMEREN VON AGGREGATEN DAVON

CHARGE DE CHROMATOGRAPHIE À ÉCHANGE DE CATIONS PERMETTANT DE RAFFINER DES ANTICORPS, ET PROCÉDÉ DE SÉPARATION DE MONOMÈRES D'ANTICORPS À PARTIR DE LEURS AGGREGATES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority:  **18.10.2012   JP 2012230396**

(43) Date of publication of application:
**26.08.2015   Bulletin 2015/35**

(73) Proprietor: **JNC Corporation**
**Chiyoda-ku**
**Tokyo 100-8105 (JP)**

(72) Inventors:
- **ISHIHARA, Takashi**
  **Tokyo 100-8185 (JP)**
- **SUZAWA, Toshiyuki**
  **Tokyo 100-8185 (JP)**
- **SUZUKI, Yasuaki**
  **Tokyo 100-8185 (JP)**

- **MATSUMOTO, Yoshihiro**
  **Minamata-shi, Kumamoto 867-0053 (JP)**
- **AOYAMA, Shigeyuki**
  **Tokyo 100-8105 (JP)**

(74) Representative: **Mewburn Ellis LLP**
**Aurora Building**
**Counterslip**
**Bristol BS1 6BX (GB)**

(56) References cited:
**EP-A1- 0 043 159        EP-A1- 1 448 242**
**WO-A1-97/18024        WO-A1-2012/015379**
**JP-A- 2012 032 392      US-A- 5 445 732**
**US-A1- 2002 017 149    US-A1- 2005 181 378**
**US-A1- 2011 301 330    US-A1- 2012 029 176**

- **None**

**Description**

**Technical Field**

The present invention relates to a cation exchange chromatography media used in an antibody drug purifying step, and a method for separating an antibody monomer from aggregates thereof to be produced in an antibody drug manufacturing process.

**Background Art**

Purifying a biomedicine applying chromatography is widely known, and separation of impurities from an object is performed utilizing various kinds of intermolecular interactions. Specific examples include a separation method utilizing an electrostatic interaction in ion exchange chromatography, a separation method utilizing a hydrophobic interaction in hydrophobic chromatography, and a separation method utilizing an affinity interaction to an antibody, such as protein A chromatography.

In manufacture of an antibody drug, provision of a product with high purity is important, and a purifying process thereof is applied in a combination of the protein A chromatography, and cation exchange chromatography, anion exchange chromatography and so forth. Above all, antibody aggregates being one of the impurities are produced in a culture or purifing process, and considered to cause a decrease in antigen recognition ability or to cause a side effect, and elimination thereof becomes an important control item in antibody drug manufacture.

In Biotechnology and Bioengineering 108, 1494-1508, 2011 (Non-patent literature No. 1), a mechanism of forming the antibodyaggregates , importance of aggregates elimination or the like is reported. However, a problem exists in which elimination of the antibody aggregates is quite difficult in an affinity purifying process by protein A chromatography. Therefore, separation of the antibody from the aggregates to obtain the antibody with high purity in a process in and after a purifying process by the protein A chromatography has become an important issue for an antibody drug manufacturer.

For such a purpose, for example, a proposal has been made on separation of an antibody from aggregates thereof using a chromatography media having a mixed mode ligand in Non-patent literature No. 1. Moreover, a proposal has been made on separation of an antibody from aggregates thereof using a hydrophobic (phenyl) chromatography media in J. Chromatography A, 1217, 216-224, 2010 (Non-patent literature No. 2) and J. Chromatography A, 1216, 902-909, 2009 (Non-patent literature No. 3). However, a separation method using the media has many practical issues such as low capacity of adsorbing the antibody being an object and use under a high salt concentration.

The cation exchange chromatography is applied for the purpose of elimination of the antibodyaggregates , elimination of protein derived from a host or elimination of a protein A leak, is inexpensive and has high use performance in manufacture of many bio preparations to be a significantly important processin the purifying process in and after the protein A chromatography. Therefore, the impurities such as the antibody aggregates and the protein derived from the host as occurred in an antibody drug manufacturing process are deemed to be preferably reduced as much as possible by cation exchange chromatographyprocess .

As adaptation of the cation exchange chromatography to purifying the antibody, for example, WO 2010/127069 A (Patent literature No. 1) discloses purifying IGF1R (Insulin-like Growth Factor-1 Receptor), and use of a cation exchanger effective in eliminating impurities such as the antibodyaggregates .

However, in use thereof in the antibody drug manufacture, the cation exchange chromatography media is required to have not only performance of separation of a product from the impurities, but also from a viewpoint of productivity, a high amount of adsorption of the antibody, and also capability of treatment at a high flow rate without compression.

As a cation exchange chromatography media having high adsorption capacity for purifying the protein, Capto (registered trademark) S (made by GE Healthcare Ltd.), TOYOPEARL (registered trademark) GigaCap S (made by Tosoh Corporation), Fractogel (registered trademark) SE Hicap, Eshmuno (registered trademark) S (all, made by Merck Ltd.), UNOsphere (registered trademark) S (made by Bio-Rad Laboratories, Inc.) or POROS (registered trademark) XS (Applied Biosystems, Inc.) has been recently developed.

For example, JP H1-310744 A (Patent literature No. 2) discloses a method for manufacturing an ion exchange chromatography media having a graft polymer as a ligand. Here, description is made on cation exchange chromatography and anion exchange chromatography using as a base carrier Fractogel (registered trademark) TSK HW 65 (S) or LiChrospher (registered trademark) diol, and also a method for manufacturing a chromatography media having a copolymer as a ligand. However, no study has been conducted on separation of the antibody from the aggregates in examples of the present report.

As an example of cation exchange chromatography media having as a ligand a graft polymer other than the media described above, JP 2011-529508 A (Patent literature No. 3) discloses a method for introducing as a ligand a copolymer by graft polymerization into Fractogel (registered trademark) TSK HW 65(M). The patent describes that dynamic binding capacity of a polyclonal antibody as measured for 2 minutes as contact time becomes higher by copolymerizing a monomer containing sulfonic acid and a monomer containing no sulfonic acid in comparison with a case where only the monomer containing sulfonic acid is subjected to graft polymerization. However, the patent makes no clear description on a relationship between a composition of the copolymer and the dynamic binding capacity. Moreover, the patent describes neither recognition of importance of influence of a composition of a strong cation group and a neutral group of the copolymer on separation of the antibody from the aggregates nor study thereon.

JP 2010-528271 A (Patent literature No. 4) discloses a cation exchange chromatography carrier in which a graft polymer having both an ion exchange group and a hydrophobic group is introduced as a ligand into Fractogel (registered trademark) TSK HW 65(M) by using as a ligand a monomer having a hydrophobic interaction. The patent describes indication of a dynamic binding capacity of 76.1 mg/mL in a monoclonal antibody even under conditions of NaCl having a concentration of 150 mM by using the chromatography media (Table 3). However, no study has been conducted herein on separation of the antibody from the aggregates, either.

On the other hand, WO 2007/123242 A (Patent literature No. 5) describes that a media formed by bonding as a ligand polyacrylic acid with a base media prepared using as a raw material a methacrylic polymer is further excellent in separation of an antibody monomer from aggregates thereof in comparison with a media prepared by directly bonding a carboxymethyl group with the base carrier. However, when the media is manufactured, synthesis of polyacrylic acid in advance is required, and thus such an art has a possibility of no respond to an issue of providing an inexpensive chromatography media desired by the antibody drug manufacturer. Moreover, no study has been made on other important characteristics of the chromatography media , such as the antibody binding capacity, and thus an issue remains in view of practicality.

As described above, a cation exchange chromatography media that allows efficient separation of the antibody monomer from the aggregates and has as a ligand a graft polymer having high adsorptivity is not known.

## Citation List

### Patent Literature

    Patent literature No. 1: WO 2010/127069 A.
    Patent literature No. 2: JP H1-310744 A.
    Patent literature No. 3: JP 2011-529508 A.
    Patent literature No. 4: JP 2010-528271 A.
    Patent literature No. 5: WO 2007/123242 A.

### Non-patent Literature

    Non-patent literature No. 1: Biotechnology and Bioengineering 108, 1494-1508, 2011.
    Non-patent literature No. 2: J. Chromatography A, 1217, 216-224, 2010.
    Non-patent literature No. 3: J. Chromatography A, 1216, 902-909, 2009.

## Summary of Invention

### Technical Problem

Under such a situation, desire has been expressed for provision of an ion exchange chromatography media that can be preferably used for purifying an antibody drug, in particular, provision of a cation exchange chromatography media in which separation performance of an antibody monomer from an antibody aggregates being aggregates thereof to be produced in an antibody drug manufacturing process is satisfactory, and antibody adsorptivity is high, and the antibody monomer can be obtained with high purity.

### Solution to Problem

The present inventors have diligently continued to conduct study in order to solve the problem described above. As a result, the present inventors have found that a structure formed by polymerizing a monomer containing at least one sulfone group at a specific composition is bonded as a ligand on porous particles to allow efficient separation of an antibody monomer from aggregates thereof, and allows provision of a cation exchange chromatography media having high antibody adsorptivity, and thus have completed the invention.

More specifically, the invention concerns a cation exchange chromatography media for purifying of an antibody, and a method for separating an antibody monomer from aggregates thereof, and so forth as defined in the claims.

**Advantageous Effects of Invention**

A cation exchange chromatography media according to the invention can be preferably used for purifying an antibody drug. According to a preferred embodiment of the invention, the cation exchange chromatography media according to the invention is used to allow efficient separation of an antibody monomer from aggregates thereof to be produced in an antibody drug manufacturing process, and therefore the antibody monomer can be obtained with high purity.

**Brief Description of Drawings**

Figure 1 is a chromatogram showing measurement results of degree of separation ($\Delta$C) of a monoclonal antibody by a gel in Example 1.
Figure 2 is a chromatogram showing measurement results of degree of separation ($\Delta$C) of a monoclonal antibody by a gel in Example 2.

**Description of Embodiments**

A cation exchange chromatography media for purifying an antibody, a method for separating an antibody monomer from aggregates thereof using the cation exchange chromatographymedia , and so forth according to the invention will be described in detail below.

(1) Cation exchange chromatography media for purifying antibody The cation exchange chromatography media for purifying the antibody according to the invention has structure formed by bonding a base media containing porous particles with a polymer composed of, in the range
of 30 to less than 100 mol% based on the total monomer, **2-acrylamide-2-methylpropanesulfonic acid, as defined in the claims, wherein ion exchange capacity thereof is from 60 to 300 $\mu$mol/mL.**

(1.1) Porous particles being base carrier

In the cation exchange chromatography media according to the disclosure, the porous particles used as the base media are not particularly limited, if the particles have a functional group (for example, a hydroxy group or a carbamoyl group) for introducing a strong cation monomer unit, and when necessary, a neutral monomer unit and a weak cation monomer unit thereinto. Specific examples of such porous particles preferably include a compound having the functional group as described above, such as polysaccharides including agarose, dextran, starch, cellulose, pullulan, chitin, chitosan, cellulose triacetate and diacetyl cellulose and a derivative thereof; and an organic polymer including polyacrylamide, polymethacrylamide, polyacrylate, polymethacrylate, polyalkylvinylether and polyvinylalcohol. The porous particles can secure mechanical strength, and thus preferably forms crosslinked structure. In the invention, crosslinked cellulose particles in which a skeleton of the cellulose particles is reinforced by a crosslinking reaction is used.

The crosslinked cellulose particles used in the invention are not particularly limited, if the particles are ordinarily used as the base media of chromatography. Cellulose as a raw material may be crystalline cellulose or amorphous cellulose, but is preferably crystalline cellulose due to high strength.

Specific examples of the crosslinked cellulose particles that can be preferably used in the invention include a porous cellulose gel disclosed in JP 2009-242770 A. The crosslinked cellulose particles obtained by the method disclosed in the patent, more specifically, the method for crosslinking the particles under continuous dropwise addition or divided addition, over 3 hours or more, of a crosslinking agent in an amount of 4 to 12 times the number of moles of a cellulose monomer and alkali in an amount of 0.1 to 1.5 times the number of moles of the crosslinking agent in the presence of at least one kind of inorganic salt selected from a group of hydrochloride, sulfate, phosphate and borate in an amount of 6 to 20 times the number of moles of the cellulose monomer to a suspension of non-crosslinked cellulose particles have high mechanical strength and can be used at a high flow rate to give the cation exchange chromatography media with high productivity. Here, "cellulose monomer" means a glucose unit being a constitutional unit of cellulose, and the number of moles (more specifically, polymerization degree) of the cellulose monomer is calculated from dry weight of cellulose by defining an amount obtained by subtracting moisture from glucose 1 unit, more specifically, molecular weight of 162 taken as 1 mol.

A shape of the crosslinked cellulose particles is not particularly limited, but is preferably spherical in view of high mechanical strength, excellent gel sedimentation properties and capability of preparation of a uniform packed bed. In the above case, sphericity of the crosslinked cellulose particles is preferably 0.8 to 1.0. Here, "sphericity" means a ratio of minor axis to major axis (minor axis/major axis) of the cellulose particles.

Spherical cellulose particles can be easily obtained, for example, by dissolving crystalline cellulose or cellulose having a crystalline region and an amorphous region to regenerate the cellulose. Specific examples of a method for manufacturing the spherical cellulose include a method through acetate ester as described in JP S55-39565 B and JP S55-40618 B, a method for granulating the cellulose from a solution using calcium thiocyanate as described in JP S63-62252 B, a method for manufacturing the cellulose from a paraformaldehyde-dimethylsulfoxide solution as described in JP S59-38203 A, and a method for forming the cellulose from a cellulose solution in which the cellulose is dissolved into lithium chloride-containing amide as described in JP 3663666 B. Moreover, spherical crosslinked cellulose particles can be obtained by crosslinking the spherical cellulose particles.

A particle size of the porous particles used in the invention is preferably 10 to 500 micrometers, further preferably, 30 to 200 micrometers, and particularly preferably, 50 to 150 micrometers. Moreover, a mean particle size is preferably 30 to 1,000 micrometers, further preferably, 40 to 200 micrometers, and still further preferably, 50 to 100 micrometers.

In addition, the particle size and the mean particle size of the porous particles herein are calculated using a laser diffraction scattering particle size distribution analyzer having as a measurement principle a method for determining a particle size distribution by calculation from a diffracted and scattered light intensity distribution pattern emitted from a group of particles after the group of particles is irradiated with a laser beam. As the analyzer, Laser Diffraction Scattering Particle Size Distribution Analyzer LA-950 made by HORIBA, Ltd. or the like can be used.

Alternatively, a particle size in an image photographed by an optical microscope is measured using a caliper or the like, and thus an original particle size can be determined from photographing magnification. Then, from a value of each particle size determined from an optical microscope photograph, a mean particle size can be calculated by a formula described below.

$$\text{Volume mean particle size } (M_V) = \Sigma(nd^4) \; / \; \Sigma(nd^3)$$

(In the formula, nd represents the value of each particle size determined from the optical microscope photograph, and n represents the number of measured particles.)

Porosity of the porous particles used as the base media in the invention can be featured by characteristics of a pore size. One of indices indicating pore size characteristics includes a gel partition coefficient Kav. From a relation with physical strength of particles or diffusibility in particles of a target material serving as a refining object, the pore size influences flow rate characteristics or dynamic binding capacity of the media. Therefore, an optimum design according to a purpose is required. From a viewpoint of the dynamic binding capacity, particularly, as the pore size of the porous particles used in the invention, the gel partition coefficient Kav when pure water is used as a mobile phase in standard polyethylene oxide of weight-average molecular weight of $1.5 \times 10^5$ Da is in the range of 0.15 to 0.6, further preferably, in the range of 0.2 to 0.55, and still further preferably, in the range of 0.3 to 0.5.

The gel partition coefficient Kav can be determined from a relationship between elution volume and column volume of a standard reference material (polyethylene oxide, for example) having specific molecular weight according to the following formula:

$$Kav = (Ve - V_0) \; / \; (Vt - V_0).$$

(In the formula, Ve represents retention volume (mL) of a sample, Vt represents empty column volume (mL), and $V_0$ represents blue dextran retention volume (mL).)

A method for measuring the gel partition coefficient Kav is described in Seibutsukagaku Jikkenho (Biochemistry Experimental Method) 2 "Gel chromatography," first edition, authored by L. Fischer (Tokyo Kagaku Dojin), for example. The gel partition coefficient Kav of the crosslinked porous cellulose particles used in the invention can be adjusted by controlling a dissolved concentration of cellulose during forming the particles, for example.

Into the porous particles used as the base media in the invention, if the amount is within the range in which an object and the operation and effect of the invention are not adversely affected, an arbitrary substituent such as a hydrophobic group including a phenyl group and a butyl group may be introduced thereinto as well. When the hydrophobic group is introduced into the porous particles, the hydrophobic group can be introduced thereinto by allowing phenyl glycidyl ether, butyl glycidyl ether or the like to react with the particles in an alkaline solution.

(1.2) Polymer as ligand

In the cation exchange chromatography media for purifying the antibody according to the invention, with the porous particles described above, as the ligand, the polymer containing, in the range of 30 to less than 100 mol% based on the total monomer, the strong cation monomer 2-acrylamide-2-methylpropanesulfonic acid is bonded.

The ion exchange capacity of the cation exchange is 60 $\mu$mol/mL or more and 300 $\mu$mol/mL or less. When the capacity is within the range, performance of separation of the antibody monomer from the aggregates thereof becomes satisfactory.

In addition, the ion exchange capacity herein can be generally determined by acid-base titration as described in WO 2007/027139 A or back titration as described in Journal of Chromatography A, 1146, 202-215, for example.

The polymer further contains the neutral monomer unit **N,N-dimethylacrylamide or the weak cation monomer unit acrylic acid.**

The polymer used as the ligand in the cation exchange chromatography media according to the invention contains the neutral monomer unit and/or the weak cation monomer unit, with the result that the polymer can provide the media with further preferred characteristics.

For example, in the cation exchange chromatography media prepared by bonding as the ligand a copolymer of 2-acrylamide-2-methylpropanesulfonic acid and N,N-dimethylacrylamide, an effect on an increase in an amount of adsorption, particularly, the dynamic binding capacity can be expected. Moreover, the cation exchange chromatography media is particularly excellent in the performance of separation of the antibody monomer from the aggregates thereof, and can efficiently separate and purify the antibody monomer.

Moreover, in the cation exchange chromatography media prepared by bonding as the base media a copolymer composed of the strong cation monomer unit 2-acrylamide-2-methylpropanesulfonic acid and the weak cation monomer unit acrylic acid, the effect on the increase in the amount of adsorption, particularly the dynamic adsorption capacity can be expected, and also is excellent in the performance of separation of the antibody monomer from the aggregates thereof. The weak cation monomer can also function as a ligand of a weak cation exchanger generally utilized in a field of purifying of protein, and in the cation exchange chromatography media according to the invention, an effect on eliminating impurities derived from a host, or the like can be expected by an effect of a weak cation group.

When the polymer is a copolymer composed of the strong cation monomer unit 2-acrylamide-2-methylpropanesulfonic acid and the neutral monomer unit N,N-dimethylacrylamide, a ratio of the strong cation monomer unit based on the total monomer composing the copolymer is 30 mol% or more, preferably, 40 mol% or more, and further preferably, 50 mol% or more. Moreover, the ratio of the strong cation monomer unit is less than 100 mol%.

Further, when the dynamic binding capacity is taken into consideration, the ion exchange capacity is 60 $\mu$mol/mL or more, preferably, 70 $\mu$mol/mL or more, further preferably, 90 $\mu$mol/mL or more, and particularly preferably, 100 $\mu$mol/mL or more. Moreover, the ion exchange capacity is 300 $\mu$mol/mL or less, preferably, 250 $\mu$mol/mL or less, further preferably, 210 $\mu$mol/mL or less, and particularly preferably, 150 $\mu$mol/mL or less.

In an optimum embodiment, the ratio of the strong cation monomer unit is 40 mol% or more and less than 100 mol%, and the ion exchange capacity is 60 to 150 $\mu$mol/mL.

When the polymer is a copolymer composed of the strong cation monomer unit 2-acrylamide-2-methylpropanesulfonic acid and the weak cation monomer unit acrylic acid, a ratio of the strong cation monomer unit based on the total monomer composing the copolymer is 30 mol% or more, preferably, 40 mol% or more, and further preferably, 50 mol% or more. Moreover, the ratio of the strong cation monomer unit is less than 100 mol%.

Further, when the dynamic binding capacity is taken into consideration, the ion exchange capacity is 60 $\mu$mol/mL or more, preferably, 70 $\mu$mol/mL or more, and further preferably, 90 $\mu$mol/mL or more. Moreover, the ion exchange capacity is 300 $\mu$mol/mL or less, and preferably, 250 $\mu$mol/mL or less.

In the optimum embodiment, the ratio of the strong cation monomer unit is 50 mol% or more and less than 100 mol%, and the ion exchange capacity is 90 to 250 $\mu$mol/mL.

In addition, the ratio (S density) of the strong cation monomer unit herein can be obtained by calculating a mole ratio of S to N or Na contained in a monomer unit by using a measured S content, N content or Na content. The S content, the N content or the Na content can be measured by applying emission spectrophotometry, an elemental analysis method or atomic absorption spectrophotometry.

(1.3) Method for manufacturing cation exchange chromatography media

Next, the method for manufacturing the cation exchange chromatography media for purifying the antibody according to the invention will be described.

The cation exchange chromatography media according to the invention is manufactured by introducing the polymer into the base media containing the porous particles by a graft polymerization reaction. In the invention, the graft polymerization reaction is progressed using as a base point a radical formed by action of Ce (IV) on a hydroxy group under acid conditions. The radical serving as the base point reacts with the monomer present in a system to allow formation of a graft polymer on the basemedia .

Specifically, pure water and the strong cation monomer are first put in a reaction vessel and dissolved thereinto, and then neutralized with alkali such as a sodium hydroxide solution. To the solution, an additional monomer is added when necessary, the base media is added thereto into a slurry form. In the above state, the resulting slurry is stirred for 0.5 to 4.0 hours while nitrogen is blown into the reaction vessel. After stirring, to the reaction vessel, a solution prepared by dissolving Ce (IV) salt such as ammonium cerium sulfate into dilute nitric acid is slowly added dropwise. After dropwise addition, the resulting reaction solution is heated to the range of 10 to 70°C, and preferably, 30 to 50°C, and stirred for 6 to 40 hours. After completion of the reaction, the resulting wet gel is washed with pure water, and subsequently with dilute sulfuric acid, and then washed with pure water until the resulting solution becomes neutral, and thus a target cation exchange chromatography media can be obtained.

The reaction is preferably performed in the range of 0 to 4 of pH of the solution.

The Ce(IV) salt is preferably in the range of 0.0001 to 1 mol/mL in a mole concentration in the reaction solution, and further preferably, in the range of 0.01 to 0.1 mol/mL.

A mole ratio of charged amounts of the strong cation monomer used for the reaction to any other monomer such as the neutral monomer and the weak cation monomer is preferably in the range of 99 : 1 to 40 : 60, and further preferably, in the range of 70 : 30 to 45 : 55.

In addition, for the graft polymerization reaction using Ce(IV), Journal of Polymer Science, 1958, No. 122, 242-243 can be referred to.

(2) Separation method

The cation exchange chromatography media for purifying of the antibody according to the invention is excellent in the performance of separation of the antibody monomer from the aggregates thereof to be produced in an antibody drug manufacturing process, and therefore can be preferably used in a biomedicine purifying process or the like. Specifically, the cation exchange chromatography media can be used for the purpose of packing the cation exchange chromatography media according to the invention into a column to flow a liquid containing the object and the impurities therethrough to adsorb only the object or the impurities thereon, or for the purpose of adsorbing both the object and the impurities and stepwise or continuously increasing a salt concentration during elution to separate the object from the impurities by utilizing a difference in affinity with the chromatographymedia .

Specific examples of the antibody used in the invention include a monoclonal antibody or a polyclonal antibody. Specific examples of kinds of antibodies include a mouse antibody, a lama antibody, a chimeric antibody, a humanized antibody, a human antibody or an antibody obtained by modifying an Fc region thereof or the like. Specific examples of a molecular form include IgG, IgM, IgA, IgD, IgE, Fab, Fc, Fc-fusion protein, VH, VL, VHH, Fab'2, scFv, scFab, scDb or scDbFc.

Moreover, the antibody used in the invention also includes a monoclonal antibody or a polyclonal antibody prepared by positively modifying part of the monoclonal antibody or the polyclonal antibody. Specific examples of a method of modifying the monoclonal antibody or the polyclonal antibody include a method described in Journal of PHARMACEUTICAL SCIENCES, 2011, 100, 2104-2119.

The antibody monomer in the invention means a molecule composed of one molecule of the antibody. antibody aggregates mean molecules in which a monomer of two or more molecules of the antibody are aggregated by a covalent bond or a non-covalent bond. Specific examples include a dimer, a trimer, a multimer, an agglomerate or an aggregate.

When cation exchange chromatography using the cation exchange chromatography media according to the invention is applied, a target antibody monomer can be separated from the aggregates thereof.

Further, the cation exchange chromatography using the cation exchange chromatography media according to the invention may be used for separation of the antibody monomer from the impurities to be produced in the antibody drug manufacturing process. Specific examples of the impurities include a product produced during a culturing process or any other chromatography treatment step for a protein derived from a cultured cell, nucleic acid, a virus, a protein A leak, a decomposed product of an antibody, and a modified product of a target antibody subjected to modification, elimination, oxidization or deamidation of a sugar-chain component.

Specific examples of an antibody-containing aqueous solution provided for the cation exchange chromatography include a composition obtained from a living body, such as plasma, serum, milk or urine, or a composition obtained from a culture liquid of a cell producing an antibody obtained by applying a gene recombinant technology or a cell fusion technology, or bacteria such as Escherichia coli, or a transgenic nonhuman animal, a plant or an insect.

Specific examples of the cell producing the antibody include a transformant in which a gene encoding a desired antibody is incorporated in a host cell. Specific examples of the host cell include a cell strain of an animal cell, a plant cell or a yeast cell. Specific examples include a Chinese hamster ovary cell (CHO cell), an NS0 cell being a mouse myeloma cell, an SP2/0 cell, a YB2/0 cell being a rat myeloma cell, an IR983F cell, a BHK cell being a cell derived from a Syrian hamster kidney, a Namalwa cell being a human myeloma cell, an embryonic stem cell or an amphicytula.

As a medium for culturing the cell producing the antibody, any medium is used, if the medium is suitable for culture of each cell. For example, as a medium for culturing the animal cell, the medium used for culture of an ordinary animal cell is used. For example, any medium such as a serum-containing medium, an animal component-free medium, such as a serum albumin or a serum fraction, a serum-free medium or a protein-free medium is used, but a serum-free medium or a protein-free medium is preferably used. Moreover, when necessary, a physiologically active substance, a nutritional factor or the like required for growth of the cell producing the antibody can be added thereto. The above additives are incorporated into the medium in advance before culture, or appropriately additionally supplied into the culture liquid as an additional medium or an additional solution during culture. As a method of additionally supplying the additive, any form may be applied such as one solution or a mixed solution of two or more kinds, and an addition method may be either continuous or intermittent.

Specific examples of the transgenic nonhuman animal, the plant or the insect producing the antibody include a nonhuman animal, a plant or an insect in which a gene encoding a protein is incorporated into a cell. Specific examples of the nonhuman animal include a mouse, a rat, a guinea pig, a hamster, a rabbit, a dog, sheep, a pig, a goat, cattle or a monkey. Specific examples of the plant include tobacco, potato, tomato, carrot, soybean, oilseed rape, alfalfa, rice, wheat, barley or cone.

Moreover, specific examples of the antibody-containing aqueous solution provided for the cation exchange chromatography in the invention include a solution obtained from a living body, such as the antibody-containing plasma or urine as described above, and also an antibody-containing aqueous solution obtained in a purifying step. Specific examples include a cell elimination liquid, a deposit elimination liquid, an alcohol fraction liquid, a curing salting fraction liquid and a chromatography eluate. Further, when insoluble matters such as particles exist, the antibody-containing aqueous solution may be provided for the purifying method of the invention after insoluble matters are eliminated in advance or after the insoluble matters are eliminated. Specific examples of a method for eliminating the insoluble matters such as the particles include a centrifugal separation method, a cross-flow filtration method (tangential-flow filtration method), a filtration method using a depth filter, a filtration method using a membrane filter or dialysis, or a method in combination thereof.

Moreover, when necessary, for example, an ultrafiltration method using an ultrafiltration membrane is applied to adjust under preferred conditions pH, electric conductivity, a buffer solution, a salt concentration, an additive or an antibody concentration of the antibody-containing aqueous solution, an antibody load per unit volume of the cation exchange chromatographymedia , or the like, and then the antibody-containing aqueous solution is provided for the cation exchange chromatography.

Specific examples of a method for adjusting the pH, the electric conductivity, the buffer solution, the salt concentration, the additive or the antibody concentration of the antibody-containing aqueous solution, or the antibody load per unit volume of the cation exchange chromatography media include an ultrafiltration method using an ultrafiltration membrane.

The cation exchange chromatography is applied thereto in an adsorption mode or a non-adsorption mode according to the purpose thereof. The adsorption mode in the cation exchange chromatography means a mode in which the antibody-containing aqueous solution provided for the cation exchange chromatography is brought into contact with the media to adsorb the target antibody on themedia , and then, when necessary, the resulting antibody is washed, and then the antibody is eluted with a buffer solution prepared by changing pH, electric conductivity, a buffer constituent, a salt concentration, an additive or the like to collect fractions adsorbed onto themedia . The non-adsorption mode in the cation exchange chromatography means a mode in which the antibody-containing aqueous solution provided for the cation exchange chromatography is brought into contact with the media to collect fractions non-adsorbed onto themedia . On the above occasion, the target antibody monomer can be separated from a compound that is desired to be separated by collecting suitable fractions. In the buffer solution used for washing or elution, preferred conditions are selected for each of the pH, the electric conductivity, the buffer constituent, the salt concentration, the additive or the like.

When chromatography conditions are selected, a difference in the affinity with the chromatography media between the target antibody monomer and the compound that is desired to be separated is utilized. For example, the conditions are set up in consideration of a difference in media structure (ligand species, ligand density, ligand orientation, a particle size, a pore size, a base media composition or the like) and physical and chemical properties (an isoelectric point, electric charge, a degree of hydrophobicity, a molecular size, steric structure or the like) between the object and the compound that is desired to be separated.

Specific examples of an elution method in the adsorption mode include a one-step elution method to allow elution by passing a buffer solution having a specific salt concentration or pH so as to cause a decrease in the affinity between the target antibody monomer and the media, a stepwise method to allow elution of the target antibody monomer by stepwise changing the salt concentration or the pH, or a gradient method to allow elution of the target antibody monomer by continuously changing the salt concentration or the pH.

A component in the buffer solution used for the antibody-containing aqueous solution and washing or elution is not particularly limited, if the component has buffering ability. Specific examples include 1 to 300 mmol/L phosphate, citrate, acetate, succinate, maleate, borate, Tris (base), HEPES, MES, PIPES, MOPS, TES or Tricine. Moreover, the salt described above can also be used, for example, in combination with any other salt such as sodium chloride, potassium chloride, calcium chloride, sodium citrate, sodium sulfate or ammonium sulfate. Further, the component in the buffer solution can also be used in combination with amino acid such as glycine, alanine, arginine, serine, threonine, glutamic acid, aspartic acid or histidine, sugar such as glucose, sucrose, lactose or sialic acid, or a derivative thereof, for example.

Then, pH of the buffer solution used for the antibody-containing aqueous solution and washing or elution is preferably in the range of 2 to 9, and further preferably, in the range of 3 to 8.

Linear velocity of the buffer solution used for the antibody-containing aqueous solution and washing or elution is preferably in the range of 50 to 1,000 cm/h.

The antibody load per unit volume of the cation exchange chromatography media is preferably in the range of 10 to 200 g/L, and further preferably, in the range of 60 to 150 g/L.

In antibody drug manufacture, any other purifyng method may be further applied in combination with the cation exchange chromatography using the cation exchange chromatography media according to the invention. As the purifying method in combination with the cation exchange chromatography, any method is applied, if the purifying method is suitable for drug manufacture. Specific examples include chromatography, activated carbon treatment, alcohol fractionation, a deposit elimination method, curing salting, exchanging of buffer solutions, concentration, dilution, filtration, viral inactivation and virus elimination. As the purifying method in combination with the cation exchange chromatography, a plurality of kinds or numbers may be combined therewith. Moreover, the purifying method in combination with the cation exchange chromatography can be applied without regard to application before or after the cation exchange chromatography.

Specific examples of the carrier or a membrane used for chromatography in combination with the cation exchange chromatography include an affinity media such as a heparin media or a protein Amedia, a cation exchangemedia, a cation exchange membrane, an anion exchangemedia, an anion exchange membrane, a gel filtrationmedia, a hydrophobic interactionmedia, a reverse phasemedia, a hydroxyapatitemedia, a fluoroapatitemedia, a sulfated cellulosemedia, a sulfated agarose media and a mixed mode (multi-modal)media.

In characteristics of the cation exchange chromatography media according to the invention, as one of indices indicating characteristics of separation of the antibody monomer from the aggregates thereof, specific examples include a degree of separation $\Delta C$ using a monoclonal antibody or a degree of separation $\Delta Cp$ using a polyclonal antibody.

The degree of separation $\Delta C$ or $\Delta Cp$ can be determined by adding a solution containing the monoclonal antibody or the polyclonal antibody containing the aggregates to a column packed with the cation exchange chromatography media to monitor a peak of the monomer or the media in the eluate to be taken as a difference of electric conductivity values corresponding to the resulting peak top of the monomer or the aggregates. As the value of $\Delta C$ or $\Delta Cp$ is larger, ability of separation of the antibody monomer from the aggregates thereof is higher.

Moreover, as one of indices indicating adsorptivity, specific examples include a dynamic binding capacity (DBC) at 10%. The dynamic bonding capacity at 10% is determined by adding a solution containing an antibody having a known concentration to a column packed with the cation exchange chromatography media to monitor an absorbance of an eluate and to calculate the capacity from an amount of added protein at a time point at which 10% breakthrough in the absorbance of an added sample is observed. As the value of the dynamic binding capacity at 10% is larger, the adsorptivity of the antibody is higher.

## Examples

The invention will be described in detail by way of Examples below, but the invention is not limited to the Examples. In addition, ion exchange capacity, dynamic binding capacity at 10% of a polyclonal antibody, an S content, an N content, a Na content and degrees of separation $\Delta C$ and $\Delta Cp$ in the Examples and Comparative Examples were measured by methods described in measurement methods 1 to 7.

**Reference Example 1**

**Manufacture of 6% spherical cellulose particles (hydrous)**

(1) To 100 g of 60 wt% calcium thiocyanate aqueous solution, 6.4 g of crystalline cellulose (trade name: Ceolus PH101, made by Asahi Kasei Chemicals Corporation) was added, and the resulting mixture was heated at 110 to 120°C and dissolved with each other.

(2) As a surfactant, 6 g of sorbitan monooleate was added to the resulting solution, added dropwise to 480 mL of o-dichlorobenzene preheated at 130 to 140°C, and the resulting mixture was stirred at 200 to 300 rpm and dispersed with each other.

(3) Next, the resulting dispersion was cooled to 40°C or lower, and poured into 190 mL of methanol to obtain a suspension of particles.

(4) The suspension was filtered and fractionated, and particles were washed with 190 mL of methanol, and then subjected to filtration and fractionation. The above washing operation was performed several times.

(5) After the particles were further washed with a plenty of water, spherical cellulose particles were obtained.

(6) Next, the spherical cellulose particles were sieved through a sieve having 53 $\mu$m to 125 $\mu$m according to a JIS standard sieve specification to adjust the size to a desired particle size (actual particle size interval: 50 to 150 $\mu$m, mean particle size: about 100 $\mu$m) to obtain target 6% spherical cellulose particles (hydrous: cellulose dissolved concentration: 6%). In addition, with regard to the mean particle size herein, a particle size in an image photographed by an optical microscope was measured using a caliper or the like to determine an original particle size from photographing magnification, and the mean particle size was calculated and determined from a value of each particle size according to the following formula:

$$\text{Volume mean particle size } (M_V) = \Sigma(nd^4) / \Sigma(nd^3)$$

(In the formula, nd represents the value of each particle size determined from the optical microscope photograph, and n represents the number of measured particles.)

**Preparation of crosslinked 6% cellulose particles**

(1) To 100 g of 6% spherical cellulose particles (hydrous) obtained as described above, 121 g of pure water was added, the resulting mixture was warmed while the mixture was stirred. When temperature reached 30°C, 3.3 g of 45 wt% NaOH aqueous solution and 5.5 g of $NaBH_4$ were added thereto, and the resulting mixture was stirred. An initial alkali concentration was 0.69% (w/w).

(2) After 30 minutes, 60 g of $Na_2SO_4$ was added to the resulting reaction solution, and dissolved with each other. At the time point at which temperature reached 50°C, stirring was continued for two hours.

(3) While stirring of the resulting mixture was continued at 50°C, an amount obtained by equally dividing into 25 each of 48 g of 45 wt% NaOH aqueous solution and 50 g of epichlorohydrin was added thereto every 15 minutes over about 6 hours.

(4) After completion of addition, the resulting mixture was allowed to react with each other at a temperature of 50°C for 16 hours.

(5) After the resulting mixture was cooled to a temperature of 40°C or lower, 2.6 g of acetic acid was added thereto, and thus the resulting mixture was neutralized.

(6) The resulting reaction mixture was filtered to collect a gel, the gel was subjected to washing and filtration using pure water to obtain a target crosslinked 6% cellulose particles. On the above occasion, a mean particle size analyzed using Laser Diffraction Scattering Particle Size Distribution Analyzer LA-950 made by HORIBA, Ltd. was 85 $\mu$m. Moreover, a gel partition coefficient Kav when pure water was used as a mobile phase in standard polyethylene oxide having a weight average molecular weight of $1.5 \times 10^5$ Da was 0.38.

**Reference Example 2**

**Manufacture of 10% spherical cellulose particles (hydrous)**

(1) To 313 g of 60 wt% calcium thiocyanate aqueous solution, 34.8 g of crystalline cellulose (trade name: Ceolus PH301, made by Asahi Kasei Chemicals Corporation) was added, and the resulting mixture was heated at 110 to 120°C and dissolved with each other.

(2) As a surfactant, 1.83 g of sorbitan monooleate was added to the resulting solution, added dropwise to 480 mL of o-dichlorobenzene preheated at 130 to 140°C, and the resulting mixture was stirred at 200 to 300 rpm and dispersed with each other.

(3) Next, the resulting dispersion was cooled to 40°C or lower, and poured into 533 mL of methanol to obtain a suspension of particles.

(4) The suspension was filtered and fractionated, and particles were washed with 620 mL of methanol, and then subjected to filtration and fractionation. The above washing operation was performed several times.

(5) After the particles were further washed with a plenty of water, spherical cellulose particles were obtained.

(6) Next, the spherical cellulose particles were sieved through a sieve having 53 $\mu$m to 125 $\mu$m according to a JIS standard sieve specification to adjust the size to a desired particle size (actual particle size interval: 50 to 150 $\mu$m, mean particle size: about 100 $\mu$m) to obtain target 10% spherical cellulose particles (hydrous: cellulose dissolved concentration: 10%). The mean particle size herein was determined in a manner similar to the method described in "Manufacture of 6% spherical cellulose particles (hydrous)" in Reference Example 1.

**Preparation of crosslinked 10% cellulose particles**

(1) In 100 g of 10% spherical cellulose particles (hydrous) obtained as described above, 199 g of pure water was put, and the resulting mixture was warmed while the mixture was stirred. When temperature reached 30°C, 4.46 g of 45 wt% NaOH aqueous solution and 0.71 g of $NaBH_4$ were added thereto, and the resulting mixture was stirred. An initial alkali concentration was 0.69% (w/w).

(2) After 30 minutes, 81 g of $Na_2SO_4$ was added to the resulting reaction solution, and dissolved with each other. At the time point at which temperature reached 50°C, stirring was continued for two hours.

(3) While stirring of the resulting mixture was continued at 50°C, an amount obtained by equally dividing into 25 each of 62 g of 45 wt% NaOH aqueous solution and 64 g of epichlorohydrin was added thereto every 15 minutes over about 6 hours.

(4) After completion of addition, the resulting mixture was allowed to react with each other at a temperature of 50°C for 16 hours.

(5) After the resulting mixture was cooled to a temperature of 40°C or lower, 3.3 g of acetic acid was added thereto, and thus the resulting mixture was neutralized.

(6) The resulting reaction mixture was filtered to collect a gel, the gel was subjected to washing and filtration using pure water to obtain a target crosslinked 10% cellulose particles. On the above occasion, a mean particle size analyzed using Laser Diffraction Scattering Particle Size Distribution Analyzer LA-950 made by HORIBA, Ltd. was 93 $\mu$m. Moreover, a gel partition coefficient Kav when pure water was used as a mobile phase in standard polyethylene oxide having a weight average molecular weight of $1.5 \times 10^5$ Da was 0.27.

**Example 1**

In a 500 mL separable flask, 64.4 g of pure water and 8.7 g of 2-acrylamide-2-methylpropanesulfonic acid were added and dissolved . Next, 3.4 g of a 48.7% (w/w) sodium hydroxide aqueous solution was added thereto, and thus the resulting mixture was neutralized. Next, 80.0 g of crosslinked 6% cellulose particles in Reference Example 1 was added thereto into slurry. In the above state, the resulting mixture was stirred for 1 hour while nitrogen was blown into the separable flask. After 1 hour, a solution prepared by dissolving 5.19 g of ammonium cerium nitrate into 18.1 mL of 0.17 mol/L nitric acid was slowly added into the separable flask from a dropping funnel. After completion of dropwise addition, the resulting mixture was heated to 40°C and stirred for 22 hours under a nitrogen atmosphere. After 22 hours, stirring was stopped and the resulting slurry was subjected to suction filtration. The resulting wet gel was washed 5 times with 80 mL of pure water. Next, the gel was washed 10 times with 120 mL of 1 mol/L sulfuric acid, and then washed with pure water until a washed solution became neutral. Part of the wet gel on the above occasion was taken out, and used for measurement of ion exchange capacity. Then, the gel was washed with 160 mL of 0.5 mol/L sodium hydroxide aqueous solution. Last, the gel was washed with pure water until a washed solution became neutral. The resulting wet gel was stored in a state in which excessive moisture was eliminated. Ion exchange capacity on the above occasion was 110 $\mu$mol/mL, dynamic binding capacity at 10% of a polyclonal antibody was 61 mg/mL, an S content was 3.1% (w/w), $\Delta$C was 6.6 mS/cm and $\Delta$Cp was 26 mS/cm.

**Example 2**

In a 500 mL separable flask, 64.4 g of pure water and 8.7 g of 2-acrylamide-2-methylpropanesulfonic acid were added and dissolved . Next, 3.4 g of a 48.7% (w/w) sodium hydroxide aqueous solution was added thereto, and thus the resulting mixture was neutralized. Next, 2.3 g of N,N-dimethylacrylamide was added thereto. Next, 80.0 g of crosslinked 6% cellulose particles in Reference Example 1 was added thereto into slurry. In the above state, the resulting mixture was stirred for 1 hour while nitrogen was blown into the separable flask. After 1 hour, a solution prepared by dissolving 5.19 g of ammonium cerium nitrate into 18.1 ml of 0.17 mol/L nitric acid was slowly added into the separable flask from a dropping funnel. After completion of dropwise addition, the resulting mixture was heated to 40°C and stirred for 22 hours under a nitrogen atmosphere. After 22 hours, stirring was stopped and the resulting slurry was subjected to suction filtration. The resulting wet gel was washed 5 times with 80 mL of pure water. Next, the gel was washed 10 times with 120 mL of 1 mol/L sulfuric acid, and then washed with pure water until a washed solution became neutral. Part of the wet gel on the above occasion was taken out, and used for measurement of ion exchange capacity. Then, the gel was washed with 160 mL of 0.5 mol/L sodium hydroxide aqueous solution. Last, the gel was washed with pure water until a washed solution became neutral. The resulting wet gel was stored in a state in which excessive moisture was eliminated. Ion exchange capacity on the above occasion was 126 $\mu$mol/mL, dynamic binding capacity at 10% of a polyclonal antibody was 114 mg/mL, an S content was 2.8% (w/w), an N content was 2.1%, $\Delta$C was 6.1 mS/cm and $\Delta$Cp was 22 mS/cm.

**Example 3**

A wet gel was obtained in a manner similar to the operation in Example 2 except that an amount of N,N-dimethylacrylamide was changed to 4.5 g. Ion exchange capacity on the above occasion was 130 $\mu$mol/mL, dynamic binding capacity at 10% of a polyclonal antibody was 119 mg/mL, an S content was 2.6% (w/w), an N content was 2.9% and $\Delta$Cp was 18 mS/cm.

**Example 4**

In a 500 mL separable flask, 50.6 g of pure water and 3.0 g of 2-acrylamide-2-methylpropanesulfonic acid were added and dissolved . Next, 1.2 g of 48.7% (w/w) sodium hydroxide aqueous solution was added thereto, and thus the resulting mixture was neutralized. Next, 0.62 g of N,N-dimethylacrylamide was added thereto. Next, 60.0 g of crosslinked 6% cellulose particles in Reference Example 1 was added thereto into slurry. In the above state, the resulting mixture was stirred for 1 hour while nitrogen was blown into the separable flask. After 1 hour, a solution prepared by dissolving 3.7 g of ammonium cerium nitrate into 13.9 mL of 0.17 mol/L nitric acid was slowly added into the separable flask from a dropping funnel. After completion of dropwise addition, the resulting mixture was heated to 40°C and stirred for 22 hours under a nitrogen atmosphere. After 22 hours, stirring was stopped and the resulting slurry was subjected to suction filtration. The resulting wet gel was washed 5 times with 60 mL of pure water. Next, the gel was washed 10 times with 90 mL of 1 mol/L sulfuric acid, and then washed with pure water until a washed solution became neutral. Part of the wet gel on the above occasion was taken out, and used for measurement of ion exchange capacity. Then, the gel was washed with 120 mL of 0.5 mol/L sodium hydroxide aqueous solution. Last, the gel was washed with pure water until a washed solution became neutral. The resulting wet gel was stored in a state in which excessive moisture was eliminated. Ion exchange capacity on the above occasion was 66 $\mu$mol/mL, dynamic binding capacity at 10% of a polyclonal antibody was 73 mg/mL, an S content was 1.7% (w/w), an N content was 1.2%, $\Delta$C was 6.8 mS/cm and $\Delta$Cp was 22 mS/cm.

**Example 5**

A wet gel was obtained in a manner similar to the operation in Example 4 except that an amount of 2-acrylamide-2-methylpropanesulfonic acid was changed to 12.2 g, an amount of a 48. 7% (w/w) sodium hydroxide aqueous solution was changed to 4.85 g, and an amount of N,N-dimethylacrylamide was changed to 6.4 g. Ion exchange capacity on the above occasion was 210 $\mu$mol/mL, dynamic binding capacity at 10% of a polyclonal antibody was 101 mg/mL, an S content was 3.8% (w/w), an N content was 4.2% and $\Delta$Cp was 17 mS/cm.

**Example 6**

A wet gel was obtained in a manner similar to the operation in Example 4 except that an amount of 2-acrylamide-2-methylpropanesulfonic acid was changed to 13.1 g, an amount of a 48. 7% (w/w) sodium hydroxide aqueous solution was changed to 5.2 g, and an amount of N,N-dimethylacrylamide was changed to 5.30 g. Ion exchange capacity on the above occasion was 172 $\mu$mol/mL, dynamic binding capacity at 10% of a polyclonal antibody was 93 mg/mL, an S content was 3.2% (w/w), an N content was 3.5%, $\Delta$C was 5.8 mS/cm and $\Delta$Cp was 18 mS/cm.

**Comparative Example A**

A wet gel was obtained in a manner similar to the operation in Example 2 except that an amount of N,N-dimethylacrylamide was changed to 10.1 g. Ion exchange capacity on the above occasion was 130 $\mu$m/mL, dynamic binding capacity at 10% of a polyclonal antibody was 125 mg/mL, an S content was 2.3% (w/w), an N content was 4.1% (w/w) and $\Delta$Cp was 13 mS/cm.

**Comparative Example B**

A wet gel was obtained in a manner similar to the operation in Example 2 except that an amount of 2-acrylamide-2-methylpropanesulfonic acid was changed to 12.6 g, an amount of a 48.7% (w/w) sodium hydroxide aqueous solution was changed to 5.0 g, and an amount of charging N,N-dimethylacrylamide was changed to 23.2 g. Ion exchange capacity on the above occasion was 102 $\mu$mol/mL, dynamic binding capacity at 10% of a polyclonal antibody was 5 mg/mL, an S content was 2.6% (w/w), and an N content was 6.2% (w/w).

**Comparative Example C**

A wet gel was obtained in a manner similar to the operation in Example 4 except that an amount of 2-acrylamide-2-methylpropanesulfonic acid was changed to 18.5 g, an amount of charging a 48.7% (w/w) sodium hydroxide aqueous solution was changed to 7.35 g, and an amount of charging N,N-dimethylacrylamide was changed to 8.49 g. Ion exchange capacity on the above occasion was 318 $\mu$mol/mL, dynamic binding capacity at 10% of a polyclonal antibody was 44 mg/mL, an S content was 4.6% (w/w), an N content was 4.9% (w/w) and $\Delta$Cp was 19 mS/cm.

**Comparative Example D**

In a 500 mL separable flask, 33.3 g of pure water and 1.25 g of 2-acrylamide-2-methylpropanesulfonic acid were put and dissolved . Next, 0.49 g of 48.7% (w/w) sodium hydroxide aqueous solution was added thereto, and thus the resulting mixture was neutralized. Next, 0.14 g of acrylic acid was added thereto. Next, 40.0 g of crosslinked 6% cellulose particles in Reference Example 1 was added thereto into slurry. In the above state, the resulting mixture was stirred for 1 hour while nitrogen was blown into the separable flask. After 1 hour, a solution prepared by dissolving 2.45 g of ammonium cerium nitrate into 9.2 mL of 0.17 mol/L nitric acid was slowly added into the separable flask from a dropping funnel. After completion of dropwise addition, the resulting mixture was heated to 40°C and stirred for 22 hours under a nitrogen atmosphere. After 22 hours, stirring was stopped and the resulting slurry was subjected to suction filtration. The resulting wet gel was washed 5 times with 40 mL of pure water. Next, the gel was washed 10 times with 60 mL of 1 mol/L sulfuric acid, and then washed with pure water until a washed solution became neutral. Part of the wet gel on the above occasion was taken out, and used for measurement of ion exchange capacity. Then, the gel was washed with 80 mL of 0.5 mol/L sodium hydroxide aqueous solution. Last, the gel was washed with pure water until a washed solution became neutral. The resulting wet gel was stored in a state in which excessive moisture was eliminated. Ion exchange capacity on the above occasion was 48 $\mu$mol/mL, dynamic binding capacity at 10% of a polyclonal antibody was 45 mg/mL, an S content was 0.95% (w/w), an N content was 1.1%, $\Delta$C was 6.6 mS/cm and $\Delta$Cp was 23 mS/cm.

**Example 7**

A wet gel was obtained in a manner similar to the operation in Comparative Example D except that an amount of charging 2-acrylamide-2-methylpropanesulfonic acid was changed to 2.02 g, an amount of a 48.7% (w/w) sodium hydroxide aqueous solution was changed to 0.82 g, and an amount of acrylic acid was changed to 0.39 g. Ion exchange capacity on the above occasion was 97 $\mu$mol/mL, dynamic binding capacity at 10% of a polyclonal antibody was 77 mg/mL, an S content was 1.5% (w/w), an N content was 2.0% (w/w), $\Delta$C was 6.5 mS/cm and $\Delta$Cp was 22 mS/cm.

**Example 8**

A wet gel was obtained in a manner similar to the operation in Comparative Example D except that an amount of 2-acrylamide-2-methylpropanesulfonic acid was changed to 4.07 g, an amount of a 48.7% (w/w) sodium hydroxide aqueous solution was changed to 1.62 g, and an amount of acrylic acid was changed to 1.56 g. Ion exchange capacity on the above occasion was 246 $\mu$mol/mL, dynamic binding capacity at 10% of a polyclonal antibody was 70 mg/mL, an S content was 2.3% (w/w), an N content was 4.8% (w/w), $\Delta$C was 4.7 mS/cm and $\Delta$Cp was 19 mS/cm.

**Example 9**

In a 500 mL separable flask, 32.7 g of pure water and 2.16 g of 2-acrylamide-2-methylpropanesulfonic acid were added and dissolved . Next, 0.86 g of 48.7% (w/w) sodium hydroxide aqueous solution was added thereto, and thus the resulting mixture was neutralized. Next, 40.0 g of crosslinked 6% cellulose particles in Reference Example 1 was added thereto into slurry. In the above state, the resulting mixture was stirred for 1 hour while nitrogen was blown into the separable flask. After 1 hour, a solution prepared by dissolving 2.60 g of ammonium cerium nitrate into 9.1 mL of 0.17 mol/L nitric acid was slowly added into the separable flask from a dropping funnel. After completion of dropwise addition, the resulting mixture was heated to 40°C and stirred for 22 hours. After 22 hours, stirring was stopped and the resulting slurry was subjected to suction filtration. The resulting wet gel was washed 5 times with 40 mL of pure water. Next, the gel was washed 10 times with 60 mL of 1 mol/L sulfuric acid, and then washed with pure water until a washed solution became neutral. Part of the wet gel on the above occasion was taken out, and used for measurement of ion exchange capacity. Then, the gel was washed with 80 mL of 0.5 mol/L sodium hydroxide aqueous solution. Last, the gel was washed with pure water until a washed solution became neutral. The resulting wet gel was stored in a state in which excessive moisture was eliminated. Ion exchange capacity on the above occasion was 60 $\mu$mol/mL, dynamic binding capacity at 10% of a polyclonal antibody was 53 mg/mL, an S content was 1.6% (w/w), $\Delta$C was 6.3 mS/cm and $\Delta$Cp was 26 mS/cm.

**Example 10**

A wet gel was obtained in a manner similar to the operation in Example 1 except that an amount of 2-acrylamide-2-methylpropanesulfonic acid was changed to 4.07 g, and an amount of a 48.7% (w/w) sodium hydroxide aqueous solution was changed to 1.62 g. Ion exchange capacity on the above occasion was 240 $\mu$mol/mL, dynamic binding capacity at 10% of a polyclonal antibody was 38 mg/mL, an S content was 5.1% (w/w) and $\Delta$Cp was 25 mS/cm.

**Comparative Example E**

A wet gel was obtained in a manner similar to the operation in Example 9 except that an amount of 2-acrylamide-2-methylpropanesulfonic acid was changed to 19.4 g, and an amount of a 48.7% (w/w) sodium hydroxide aqueous solution was changed to 7.07 g. Ion exchange capacity on the above occasion was 404 $\mu$mol/mL, dynamic binding capacity at 10% of a polyclonal antibody was 26 mg/mL, an S content was 7.4% (w/w), $\Delta$C was 7.9 mS/cm and $\Delta$Cp was 25 mS/cm.

**Example 11**

In a 500 mL separable flask, 66.4 g of pure water and 7.02 g of 3-sulfopropyl methacrylate, potassium salt were added and dissolved . Next, 80.0 g of crosslinked 6% cellulose particles in Reference Example 1 was added thereto into slurry. In the above state, the resulting mixture was stirred for 1 hour while nitrogen was blown into the separable flask. After 1 hour, a solution prepared by dissolving 5.20 g of ammonium cerium nitrate into 18.1 mL of 0.17 mol/L nitric acid was slowly added into the separable flask from a dropping funnel. After completion of dropwise addition, the resulting mixture was heated to 40°C and stirred for 22 hours under a nitrogen atmosphere. After 22 hours, stirring was stopped and the resulting slurry was subjected to suction filtration. The resulting wet gel was washed 5 times with 80 mL of pure water. Next, the gel was washed 10 times with 120 mL of 1 mol/L sulfuric acid, and then washed with pure water until a washed solution became neutral. Part of the wet gel on the above occasion was taken out, and used for measurement of ion exchange capacity. Then, the gel was washed with 160 mL of 0.5 mol/L sodium hydroxide aqueous solution. Last, the gel was washed with pure water until a washed solution became neutral. The resulting wet gel was stored in a state in which excessive moisture was eliminated. Ion exchange capacity on the above occasion was 124 $\mu$mol/mL, dynamic binding capacity at 10% of a polyclonal antibody was 45 mg/mL, and $\Delta$Cp was 28 mS/cm.

**Example 12**

In a 500 mL separable flask, 91.5 g of pure water and 4.20 g of 2-acrylamide-2-methylpropanesulfonic acid were added and dissolved . Next, 1.67 g of 48.7% (w/w) sodium hydroxide aqueous solution was added thereto, and thus the resulting mixture was neutralized. Next, 50.0 g of crosslinked 10% cellulose particles in Reference Example 2 was added thereto into slurry. In the above state, the resulting mixture was stirred for 1 hour while nitrogen was blown into the separable flask. After 1 hour, a solution prepared by dissolving 5.07 g of ammonium cerium nitrate into 17.1 mL of 0.17 mol/L nitric acid was slowly added into the separable flask from a dropping funnel. After completion of dropwise addition, the resulting mixture was heated to 40°C and stirred for 22 hours under a nitrogen atmosphere. After 22 hours, stirring was stopped and the resulting slurry was subjected to suction filtration. The resulting wet gel was washed 5 times with 50 mL of pure water. Next, the gel was washed 10 times with 75 mL of 1 mol/L sulfuric acid, and then washed with pure water until a washed solution became neutral. Part of the wet gel on the above occasion was taken out, and used for measurement of ion exchange capacity. Then, the gel was washed with 100 mL of 0.5 mol/L sodium hydroxide aqueous solution. Last, the gel was washed with pure water until a washed solution became neutral. The resulting wet gel was stored in a state in which excessive moisture was eliminated. Ion exchange capacity on the above occasion was 111 $\mu$mol/mL, dynamic binding capacity at 10% of a polyclonal antibody was 38 mg/mL, an S content was 1.5% (w/w) and $\Delta$Cp was 24 mS/cm.

**Measurement method 1**

Measurement of dynamic binding capacity (DBC) at 10% using polyclonal antibody

(1) Equipment and reagent used

| | |
|---|---|
| LC system : | AKTA explorer 10S (registered trademark) |
| Buffer : | acetic acid buffer, pH 5.0, 0.05 mol/L NaCl |
| Polyclonal : antibody | $\gamma$-globulin, derived from human serum (Wako Pure Chemical Industries, Ltd.). |
| Column : | diameter 5 mm, length 5 cm |

(2) Measurement method

An antibody was dissolved into a buffer to prepare a 1 mg/mL antibody solution. Then, a column was tightly packed with a gel to which an ion exchange group was added. Next, the column was connected to a system, and the buffer was used to equilibrate the column at a flow rate of 1 mL/min until UV (ultraviolet absorbance, 280 nm), electric conductivity and pH of a column effluent became constant. Then, UV of a baseline was adjusted to zero. Next, the antibody solution was allowed to flow through the column at a flow rate of 0.5 mL/min. UV of the column effluent was monitored, and at a time point at which UV of the column effluent reached 10% of previously measured UV of the antibody solution, flow of the antibody solution was stopped. Dynamic binding capacity at 10% was determined by the following formula. the above analysis was performed in a room at 25°C.

```
{Antibody solution concentration (mg/ml) × time (min) from starting and
ending of flowing antibody solution × flow rate (mL/min) - dead volume}
/ column volume = dynamic binding capacity (mg/mL) at 10%.
```

Here, the dead volume means a volume (mL) obtained by adding a system piping volume to a column void volume.

**Measurement method 2**

Measurement of degree of separation ($\Delta$C) using monoclonal antibody containing aggregates

(1) Equipment and reagent used

| | |
|---|---|
| LC system : | AKTA explorer 10S (registered trademark) |
| A buffer : | citric acid buffer, pH 5.0 |
| B buffer : | citric acid buffer, pH 5.0, 0.5 mol/L NaCl |
| Column : | diameter 5 mm, length 5 cm |

(2) Measurement of degree of separation ($\Delta$C)

A column packed with a gel was connected to a system and equilibrated using an equilibration buffer formed of a mixed solution containing 80 vol% of A buffer and 20 vol% of B buffer. Then, 1 mL of monoclonal antibody solution was applied to the column. After completion of application, 5 column volumes of the equilibration buffer were flowed through the column. A ratio of B buffer was increased from 20 vol% to 100 vol% by applying 30 column volumes to elute an adsorbed monoclonal antibody. Elution was made at a flow rate of 0.66 mL/min for all.

(3) Method of calculation of degree of separation (ΔC)

Peaks of a monomer and aggregates were obtained by an analysis described in (2). Electric conductivity corresponding to a peak top of the monomer was taken as C1 (mS/cm), and electric conductivity corresponding to a peak top of the aggregates was taken as C2 (mS/cm), and ΔC was determined by the following formula:

$$\Delta C \ (mS/cm) = C2 \ (mS/cm) - C1 \ (mS/cm).$$

(4) Results of refinement

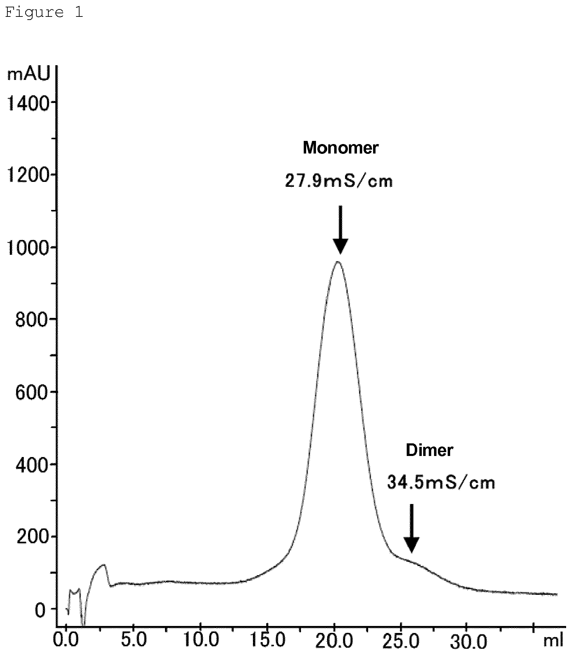

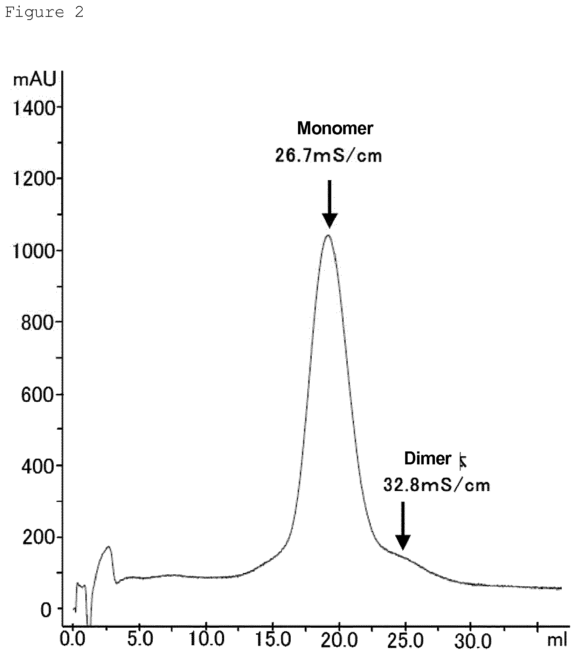

Analytical results (chromatograms) obtained by measurement of degrees of separation (ΔC) as described above for monoclonal antibodies using gels in Example 1 and Example 2 are shown in Figure 1 and Figure 2, respectively.

**Measurement method 3**

Measurement of degree of separation (ΔCp) using polyclonal antibody aggregates

(1) Equipment and reagent used

| | |
|---|---|
| LC system | : AKTA explorer 10S (registered trademark) |
| A buffer | : acetic acid buffer, pH 5.0, 0.05 mol/L NaCl |
| B buffer | : acetic acid buffer, pH 5.0, 1.0 mol/L NaCl |
| polyclonal antibody | : described in (2) |
| aggregates | : diameter 5 mm, length 5 cm |
| Column | |

(2) Method for preparing polyclonal antibody aggregates

A modified polyclonal antibody was prepared according to a method described in Journal of PHARMACEUTICAL SCIENCES, 2011, 100, 2104-2119. More specifically, 2 mg of γ-globulin, derived from human serum (Wako Pure Chemical Industries, Ltd.) was dissolved into 1 mL of 20 mmol/L phosphoric acid-citric acid buffer, pH 3.5. The resulting mixture was heated at 55°C for 15 minutes using a block heater. Then, the resulting mixture was cooled in an ice bath. PD-10 (GE Healthcare Ltd.) was used to perform buffer exchange to an acetic acid buffer, pH 5.0, 0.05 M NaCl. Formation of the modified polyclonal antibody was confirmed using gel filtration chromatography. The polyclonal antibody aggregates was stored under refrigeration before use.

(3) Measurement of degree of separation (ΔCp)

A column packed with a gel was connected to a system, and equilibrated using A buffer. A sample loop was used to apply 1 mL of polyclonal antibody aggregates solution to the column. After an unadsorbed portion was washed, a ratio of B buffer was increased from 0 vol% to 100 vol% for 120 minutes . Such operation was performed at a flow rate of 0.5 mL/min for all.

(4) Method of calculation of degree of separation (ΔCp)

Two peaks were obtained by an analysis described in (3). Electric conductivity corresponding to elution volume of a peak top of a former peak was taken as C1 (mS/cm), and electric conductivity corresponding to a peak top of a latter peak was taken as C2 (mS/cm), and ΔCp was determined by the following formula:

$$\Delta Cp \ (mS/cm) = C2 \ (mS/cm) - C1 \ (mS/cm).$$

**Measurement method 4**

Measurement of ion exchange capacity

In Examples 1 to 12 and Comparative Examples A to D, 1 mL of gel subjected to washing with sulfuric acid and then with pure water was put in a beaker, 40 mL of 0.01 mol/L sodium hydroxide solution and one drop of phenolphthalein solution were added thereto. Then, 0.1 mol/L hydrochloric acid was added to the resulting solution until a color of the solution became transparent. When an amount of hydrochloric acid added until the color became transparent was taken as A mL, ion exchange capacity (IEC) per mL of each gel can be determined by the following formula:

$$(0.01 \times 40 / 1000 - 0.1 \times A / 1000) \times 1000000 \ (\mu mol/mL).$$

In Comparative Example E, a solution was prepared by changing an amount of 0.01 mol/L sodium hydroxide solution from 40 mL to 60 mL, and applying other conditions in a manner similar to the conditions described above. Ion exchange capacity of 1 mL of gel obtained in Comparative Example E can be determined by the following formula:

$$(0.01 \times 60 / 1000 - 0.1 \times A / 1000) \times 1000000 \ (\mu mol/mL).$$

**Measurement method 5**

Measurement of S content

Gels in Examples 1 to 10 and 12 and Comparative Examples A to E were dried to determine an S content per dry weight (% by weight) by ICP (INDUCTIVELY COUPLED PLASMA) emission spectrophotometry.

**Measurement method 6**

Measurement of N content

Gels in Examples 2 to 6 and Comparative Examples A to C were dried to determine an N content per dry weight (% by weight) a CHN (Carbon Hydrogen Nitrogen) elemental analysis.

**Measurement method 7**

**Measurement of Na content**

Gels in Examples 7 and 8 and Comparative Example D were dried to determine a Na content per dry weight (% by weight) by atomic absorption spectrophotometry.

**Method for calculating S density**

(I) Examples 1 to 6 and Comparative Examples A to C

Values of an S content and an N content as measured by measurement method 5 and measurement method 6 were used to determine a ratio (S density) of a strong cation monomer unit in a copolymerization polymer from the following formula:

$$\{(S \ content \ / \ 32) \ / \ (N \ content \ / \ 14)\} \times 100 \ (\%).$$

(II) Examples 7 and 8 and Comparative Example D

Values of an S content and a Na content as measured by measurement method 5 and measurement method 7 were used to determine a ratio (S density) of a strong cation monomer unit in a copolymerization polymer from the following formula:

$$(\text{S content} / 32) / (\text{Na content} / 23) \times 100 \ (\%).$$

In addition, in the Examples described above, the monomer unit represented by formula (1) was used as the strong cation monomer, and therefore, for convenience, the ratio of the strong cation monomer unit contained in the copolymerization polymer can be roughly estimated by determining the S density represented by the above formula described.

Results in each Example and Comparative Example are presented in Tables 1 to 3.

Table 1 shows an influence of introduction of a neutral monomer on dynamic adsorption capacity and separation characteristics of a cation exchange chromatography media according to the invention.

**Table 1**

| | Strongly cationic monomer / neutral monomer | Ion exchange capacity | S content | N content | S density | Polyclonal antibody 10% DBC | ΔC | ΔCp |
|---|---|---|---|---|---|---|---|---|
| | | μmol/mL | % | % | % | mg/mL-gel | mS/cm | mS/cm |
| Example 1 | (100 : 0) | 110 | 3.1 | | 100 | 61 | 6.6 | 26 |
| Example 2 | (6 : 4) | 126 | 2.8 | 2.1 | 58 | 114 | 6.1 | 22 |
| Example 3 | (4 : 6) | 130 | 2.6 | 2.9 | 39 | 119 | - | 18 |
| Example 4 | (6 : 4) | 66 | 1.7 | 1.2 | 62 | 73 | 6.8 | 22 |
| Example 5 | (4 : 6) | 210 | 3.8 | 4.2 | 40 | 101 | - | 17 |
| Example 6 | (5 : 5) | 172 | 3.2 | 3.5 | 48 | 93 | 5.8 | 18 |
| Comparative Example A | (1 : 3) | 130 | 2.3 | 4.1 | 25 | 125 | - | 13 |
| Comparative Example B | (1 : 4) | 102 | 2.6 | 6.2 | 18 | 5 | - | - |
| Comparative Example C | (4 : 6) | 318 | 4.6 | 4.9 | 41 | 44 | - | 19 |
| Base media: Crosslinked cellulose particles. Ligand: Copolymer of strong cation monomer unit and neutral monomer unit. | | | | | | | | |

Table 2 shows an influence of introduction of a weak cation monomer on dynamic adsorption capacity and separation characteristics of a cation exchange chromatography media according to the invention.

**Table 2**

| | Ion exchange capacity | S content | Na content | S density | Polyclonal antibody 10% DBC | ΔC | ΔCp |
|---|---|---|---|---|---|---|---|
| | μmol/mL | % | % | % | mg/mL-gel | mS/cm | mS/cm |
| Example 7 | 97 | 1.5 | 2 | 54 | 77 | 6.5 | 22 |
| Example 8 | 246 | 2.3 | 4.8 | 34 | 70 | 4.7 | 19 |
| Comparative Example D | 48 | 0.95 | 1.1 | 62 | 45 | 6.6 | 23 |
| Basemedia : Crosslinked cellulose particles. Ligand: Copolymer of strong cation monomer unit and weak cation monomer unit. | | | | | | | |

Table 3 shows an influence of an amount of introduction of a strong cation monomer, a difference in structure of the strong cation monomer and a difference in Kav of crosslinked cellulose particles on dynamic adsorption capacity and separation characteristics of a cation exchange chromatography media according to the invention.

Table 3

|  | Ion exchange capacity | S content | Polyclonal antibody 10% DBC | $\Delta C$ | $\Delta Cp$ |
|---|---|---|---|---|---|
|  | $\mu$mol/mL | % | mg/mL-gel | mS/cm | mS/cm |
| Example 1 | 110 | 3.1 | 6.1 | 6.6 | 26 |
| Example 9 | 60 | 1.6 | 53 | 6.3 | 26 |
| Example 10 | 240 | 5.1 | 38 | - | 25 |
| Comparative Example E | 404 | 7.4 | 26 | 7.9 | 25 |
| Example 11 | 124 | - | 45 | - | 28 |
| Example 12 | 111 | 1.5 | 38 | - | 24 |
| Base carrier: Crosslinked cellulose particles. Ligand: Copolymer consisting of strong cation monomer unit. | | | | | |

**Industrial Applicability**

A cation exchange chromatography media for purifying of an antibody according to the invention allows effective separation of an antibody monomer from aggregates thereof produced in an antibody drug manufacturing process, and therefore can be preferably used in purifying of a biomedicine or the like.

**Claims**

1. A cation exchange chromatography media for purifying an antibody, formed by bonding a base media involving porous particles with a polymer composed of (i) in the range of 30 to less than 100 mol% based on the total monomer, 2-acrylamide-2-methylpropanesulfonic acid, and (ii) either N,N-dimethylacrylamide or acrylic acid, wherein ion exchange capacity thereof is from 60 to 300 $\mu$mol/mL; and
wherein the porous particles are crosslinked cellulose particles and have a gel partition coefficient Kav of 0.15 to 0.6 as determined when pure water is used as a mobile phase in standard polyethylene oxide having average molecular weight of 1.5 x $10^5$ Da.

2. The cation exchange chromatography media according to claim 1 in which the polymer is composed of 2-acrylamide-2-methylpropanesulfonic acid and N,N-dimethylacrylamide, wherein a ratio of the 2-acrylamide-2-methylpropanesulfonic acid monomer unit contained in the polymer is 40 mol% or more and less than 100 mol%, and ion exchange capacity thereof is 60 to 150 $\mu$mol/mL.

3. The cation exchange chromatography media according to claim 1 in which the polymer is composed of 2-acrylamide-2-methylpropanesulfonic acid and acrylic acid, wherein a ratio of the 2-acrylamide-2-methylpropanesulfonic acid monomer unit contained in the polymer is 50 mol% or more and less than 100 mol%, and ion exchange capacity thereof is 90 to 250 $\mu$mol/mL.

4. The cation exchange chromatography media according to any one of claims 1 to 3, wherein the porous particles have a gel partition coefficient Kav of 0.3 to 0.5 measured when pure water is used as a mobile phase in standard polyethylene oxide having weight average molecular weight of 1.5 $\times$ $10^5$ Da.

5. The cation exchange chromatography media according to any one of claims 1 to 4, which is for separating an antibody monomer from aggregates thereof produced in an antibody drug manufacturing process.

6. Use of the cation exchange chromatography media according to any one of the preceding claims for separating an antibody monomer from aggregates thereof produced in an antibody drug manufacturing process.

7. A method for separating an antibody monomer from aggregates thereof produced in an antibody drug manufacturing process by using the cation exchange chromatography media according to any one of items 1 to 5.

**Patentansprüche**

1.  Kationenaustauschchromatographie-Medium zum Reinigen eines Antikörpers, gebildet durch Bonden eines Basismediums, das poröse Teilchen aufweist, mit einem Polymer, das aus (i) 2-Acrylamid-2-methylpropansulfonsäure im Bereich von 30 bis weniger als 100 Mol-%, bezogen auf das gesamte Monomer, und (ii) entweder N,N-Dimethylacrylamid oder Acrylsäure besteht, wobei die Ionenaustauschkapazität davon 60 bis 300 $\mu$mol/ml beträgt; und wobei die porösen Teilchen Teilchen von vernetzter Cellulose sind und einen Gelverteilungskoeffizienten Kav von 0,15 bis 0,6 aufweisen, wie bestimmt, indem reines Wasser als mobile Phase in Standard-Polyethylenoxid mit einem mittleren Molekulargewicht von 1,5 x $10^5$ Da verwendet wird.

2.  Kationenaustauschchromatographie-Medium nach Anspruch 1, in dem das Polymer aus 2-Acrylamid-2-methylpropansulfonsäure und N,N-Dimethylacrylamid besteht, wobei ein Anteil der 2-Acrylamid-2-methylpropansulfonsäure-Monomereinheit, die in dem Polymer enthalten ist, 40 Mol-% oder mehr und weniger als 100 Mol-% beträgt und die Ionenaustauschkapazität davon 60 bis 150 $\mu$mol/ml beträgt.

3.  Kationenaustauschchromatographie-Medium nach Anspruch 1, in dem das Polymer aus 2-Acrylamid-2-methylpropansulfonsäure und Acrylsäure besteht, wobei ein Anteil der 2-Acrylamid-2-methylpropansulfonsäure-Monomereinheit, die in dem Polymer enthalten ist, 50 Mol-% oder mehr und weniger als 100 Mol-% beträgt und die Ionenaustauschkapazität davon 90 bis 250 $\mu$mol/ml beträgt.

4.  Kationenaustauschchromatographie-Medium nach einem der Ansprüche 1 bis 3, wobei die porösen Teilchen einen Gelverteilungskoeffizienten Kav von 0,3 bis 0,5 aufweisen, wie gemessen, indem reines Wasser als mobile Phase in Standard-Polyethylenoxid mit einem mittleren Molekulargewicht von 1,5 x $10^5$ Da verwendet wird.

5.  Kationenaustauschchromatographie-Medium nach einem der Ansprüche 1 bis 4, das zum Trennen eines Antikörpermonomers von Aggregaten davon, die in einem Antikörperarzneimittel-Herstellungsverfahren produziert wurden, dient.

6.  Verwendung eines Kationenaustauschchromatographie-Mediums nach einem der vorangegangenen Ansprüche zum Trennen eines Antikörpermonomers von Aggregaten davon, die in einem Antikörperarzneimittel-Herstellungsverfahren produziert wurden.

7.  Verfahren zum Trennen eines Antikörpermonomers von Aggregaten davon, die in einem Antikörperarzneimittel-Herstellungsverfahren produziert wurden, unter Verwendung eines Kationenaustauschchromatographie-Mediums nach einem der Ansprüche 1 bis 5.

**Revendications**

1.  Milieu de chromatographie par échange de cations pour purifier un anticorps, formé par liaison d'un milieu de base impliquant des particules poreuses avec un polymère composé de (i) 30 à moins de 100 % en moles, par rapport au monomère total, d'acide 2-acrylamido-2-méthylpropanesulfonique, et (ii) soit du N,N-diméthylacrylamide soit de l'acide acrylique, dans lequel la capacité d'échange d'ions de celui-ci est de 60 à 300 $\mu$mol/ml ; et
    dans lequel les particules poreuses sont des particules de cellulose réticulée et ont un coefficient de partage de la phase gel Kav de 0,15 à 0,6, tel que déterminé quand de l'eau pure est utilisée en tant que phase mobile dans un étalon de poly(oxyde d'éthylène) ayant une masse moléculaire moyenne de 1,5 x $10^5$ Da.

2.  Milieu de chromatographie par échange de cations selon la revendication 1, dans lequel le polymère est composé d'acide 2-acrylamido-2-méthylpropanesulfonique et de N,N-diméthylacrylamide, dans lequel la proportion du motif monomère acide 2-acrylamido-2-méthylpropanesulfonique contenu dans le polymère est de 40 % en moles ou plus et inférieur à 100 %, et sa capacité d'échange d'ions est de 60 à 150 $\mu$mol/ml.

3.  Milieu de chromatographie par échange de cations selon la revendication 1, dans lequel le polymère est composé d'acide 2-acrylamido-2-méthylpropanesulfonique et d'acide acrylique, dans lequel la proportion du motif monomère acide 2-acrylamido-2-méthylpropanesulfonique contenu dans le polymère est de 50 % en moles ou plus et inférieur à 100 %, et sa capacité d'échange d'ions est de 90 à 250 μmol/ml.

4. Milieu de chromatographie par échange de cations selon l'une quelconque des revendications 1 à 3, dans lequel les particules poreuses ont un coefficient de partage de la phase gel Kav de 0,3 à 0,5 mesuré quand de l'eau pure est utilisée en tant que phase mobile dans un étalon de poly(oxyde d'éthylène) ayant une masse moléculaire moyenne en masse de $1,5 \times 10^5$ Da.

5. Milieu de chromatographie par échange de cations selon l'une quelconque des revendications 1 à 4, qui est destiné à séparer un monomère d'anticorps d'avec des agrégats de celui-ci produits dans un procédé de fabrication de médicament anticorps.

6. Utilisation du milieu de chromatographie par échange de cations selon l'une quelconque des revendications précédentes pour séparer un monomère d'anticorps d'avec des agrégats de celui-ci produits dans un procédé de fabrication de médicament anticorps.

7. Procédé pour séparer monomère d'anticorps d'avec des agrégats de celui-ci produits dans un procédé de fabrication de médicament anticorps par utilisation du milieu de chromatographie par échange de cations selon l'une quelconque des revendications 1 à 5.

Figure 1

Figure 2

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2010127069 A
- JP H1310744 A
- JP 2011529508 A
- JP 2010528271 A
- WO 2007123242 A
- JP 2009242770 A
- JP S5539565 B
- JP S5540618 B
- JP S6362252 B
- JP S5938203 A
- JP 3663666 B
- WO 2007027139 A

**Non-patent literature cited in the description**

- *Biotechnology and Bioengineering,* 2011, vol. 108, 1494-1508
- *J. Chromatography A,* 2010, vol. 1217, 216-224
- *J. Chromatography A,* 2009, vol. 1216, 902-909
- Gel chromatography. **L. FISCHER.** Seibutsukagaku Jikkenho (Biochemistry Experimental Method). Tokyo Kagaku Dojin, vol. 2
- *Journal of Chromatography A,* vol. 1146, 202-215
- *Journal of Polymer Science,* 1958, 242-243
- *Journal of PHARMACEUTICAL SCIENCES,* 2011, vol. 100, 2104-2119